# EUROPÄISCHE PATENTSCHRIFT

(11) **EP 4 265 177 B1**
(45) Veröffentlichungstag und Bekanntmachung des Hinweises auf die Patenterteilung: **09.10.2024**
(21) Anmeldenummer: 22169290.8
(22) Anmeldetag: 21.04.2022
(51) Int. Cl.: A61B 5/01, A61B 5/1455, A61B 5/00

(54) **VORRICHTUNG ZUM ERFASSEN VON VITALPARAMETERN IN EINEM OHR EINES MENSCHEN UND VERFAHREN ZUM MESSEN VON VITALPARAMETERN EINES MENSCHEN**
DEVICE FOR DETECTING VITAL PARAMETERS IN A HUMAN EAR AND METHOD FOR MEASURING VITAL PARAMETERS OF A HUMAN
DISPOSITIF DE DÉTECTION DES PARAMÈTRES VITAUX DANS UN OREILLE D'UN HUMAIN ET PROCÉDÉ DE MESURE DES PARAMÈTRES VITAUX D'UN HUMAIN

(43) Veröffentlichungstag der Anmeldung: 25.10.2023
(73) Patentinhaber: Eurac Research, 39100 Bolzano (IT); Kerr S.r.l., 39100 Bolzano (IT); Minnova Med der Michaela Egebrecht, 39012 Merano (IT)
(72) Erfinder: Strapazzon, Giacomo, 39100 Bolzano (IT); Brugger, Hermann, 39100 Bolzano (IT); Masé, Michela, 39100 Bolzano (IT); Micarelli, Alessandro, 39100 Bolzano (IT); Stona, Andrea, 39100 Bolzano (IT); Egebrecht, Michaela, 39012 Merano (IT); Vinante, Mattia, 39100 Bolzano (IT); Chen, Lijun, 39100 Bolzano (IT)
(74) Vertreter: Kehl, Ascherl, Liebhoff & Ettmayr Patentanwälte Partnerschaft mbB

(56) Entgegenhaltungen:
- EP-A1- 3 020 327
- WO-A1-2017/015661
- WO-A1-2017/203251
- WO-A1-99/23941
- DE-A1- 102017 007 040

## Beschreibung

Die vorliegende Erfindung bezieht sich gemäß Anspruch 1 auf eine Vorrichtung zum Erfassen von Vitalparametern in einem Ohr eines Menschen und gemäß Anspruch 12 auf ein Verfahren zum Messen von Vitalparametern eines Menschen.

Die Druckschrift EP3020327A offenbart beispielsweise ein Ohrthermometer, welches bereits vorteilhafte Resultate liefert, da eine Abdichtung des Gehörgangs zur Umgebung bereits im Gehörgang vorgeschlagen wird, wodurch ein vorteilhaftes Klima im abgedichteten Bereich entsteht. Dennoch ist es wünschenswert noch genauere und vielfältigere Informationen über den Gesundheitszustand einer in Not geratenen Person zu erhalten. Weiter Vorrichtungen zum Erfassen von Vitalparametern im Ohr werden durch die Druckschriften WO2017/015661A1, WO99/23941A1, DE102017007040A1 und WO2017/203251A1 offenbart.

Es ist somit die Aufgabe der vorliegenden Erfindung eine genauere Erfassung des Gesundheitszustandes einer in Not geratenen Person, insbesondere unterkühlten Person, zu ermöglichen.

Die zuvor genannten Aufgabe wird erfindungsgemäß durch eine Vorrichtung zum Erfassen von zumindest einem und besonders bevorzugt zwei oder zumindest zwei Vitalparametern nach Anspruch 1 gelöst. Eine solche Vorrichtung weist bevorzugt zumindest auf: Eine optische Sensoreinrichtung zur Erfassung von mindestens einem Vitalparameter eines Lebewesens, wobei die optische Sensoreinrichtung eine Strahlungsquelle zum Emittieren von Strahlung und einen Strahlungsdetektor zum Detektieren von Strahlung aufweist, wobei die Strahlungsquelle und der Strahlungsdetektor nebeneinander, insbesondere auf einem PCB, angeordnet sind. Nebeneinander bedeutet hierbei, dass die Strahlungsquelle und der Strahlungsdetektor bevorzugt auf derselben Oberfläche und insbesondere in derselben Orientierung angeordnet sind. Weiterhin weist die Vorrichtung bevorzugt einen länglichen Einbringanteil zum Einbringen in einen Gehörgang und zum Positionieren der optischen Sensoreinrichtung in einem von Knochen umgebenen Anteil des Gehörgangs auf. Länglich bedeutet hierbei bevorzugt, dass die Erstreckung des Einbringanteils in seiner Längsrichtung ein Vielfaches größer ist als die Erstreckung in einer Richtung orthogonal zur Längsrichtung des Einbringanteils, insbesondere mindestens 3-mal solange oder mindestens 5mal solange oder mindestens 10mal solange oder z.B. bis zu 20-mal solange oder z.B. bis zu 50-mal solange ist. Die optische Sensoreinrichtung ist besonders bevorzugt an dem länglichen Einbringanteil angeordnet oder Bestandteil davon und wobei ein Temperatursensor zur Erfassung der Körperkerntemperatur an dem länglichen Einbringanteil angeordnet ist oder Bestandteil davon ist. Weiterhin ist ein mit dem Einbringanteil gekoppelter, insbesondere lösbar gekoppelter, Auswerteanteil vorgesehen, wobei der Auswerteanteil und die optische Sensoreinrichtung zumindest zeitweise, insbesondere in einem gekoppelten Zustand, signaltechnisch miteinander verbunden sind.

Diese Lösung ist vorteilhaft, da durch die Anordnung der Strahlungsquelle neben dem Strahlungsdetektor eine sehr kompakte Bauform ermöglicht wird, wodurch die optische Sensoreinrichtung sehr tief im Gehörgang positionierbar ist. Die Positionierung im Bereich des Trommelfells hat den Vorteil, dass die Vitalparameter sehr genau erfassbar sind.

Die Vorrichtung bzw. Sonde ist bevorzugt für die Verwendung bei Erwachsenen und Jugendlichen (>14 Jahre alt) geeignet. Ihre Abmessungen sind besonders bevorzugt für eine kranio-morphometrische Anatomie konzipiert, die zum Zeitpunkt der Anmeldung des vorliegenden Schutzrechts der Mehrheit der weißen kaukasischen/asiatischen Menschen entspricht, insbesondere einen darauf bezogenen definierten Gehörgang mit einer bevorzugt minimalen Länge von 12mm und einer bevorzugt maximalen Länge von 25mm.

Weitere bevorzugte Ausführungsformen der vorliegenden Erfindung sind Gegenstand der nachfolgenden Beschreibungsteile und/oder der Unteransprüche.

Die Strahlungsquelle ist gemäß der vorliegenden Erfindung auf einer Vorderseite eines Substrats zum Emittieren von Strahlung in Richtung eines Trommelfells, insbesondere in Verlängerungsrichtung des Einbringanteils, angeordnet oder ausgebildet und der Strahlungsdetektor ist besonders bevorzugt ebenfalls auf der Vorderseite des Substrats zum Detektieren von Strahlung aus Richtung des Trommelfells, insbesondere aus der Verlängerungsrichtung des Einbringanteils, angeordnet oder ausgebildet. Das Substrat ist besonders bevorzugt eine Leiterplatine bzw. ein Printed Circuit Board (PCB), wobei das PCB bevorzugt mittels Leitungen, insbesondere flexiblen Leitungen, wie z.B. Kabeln, zum Übermitteln der erfassten Signale und/oder Daten mit dem Auswerteanteil verbindbar oder verbunden ist. Bevorzugt ist zwischen der Strahlungsquelle und dem Strahlungsdetektor eine Strahlungsbarriere ausgebildet, wobei die Strahlungsquelle als zumindest zwei Leuchtmittel, insbesondere eine erste LED oder OLED und eine zweite LED oder OLED, zum Emittieren von Strahlung unterschiedlicher Wellenlängen ausgebildet ist. Als Strahlungsbarriere im Sinne der vorliegenden Erfindung wird eine Einrichtung verstanden, welche eine direkte Beleuchtung des Strahlungsdetektors mit Strahlung der Strahlungsquelle verhindert. Besonders bevorzugt ist die Strahlungsbarriere als wandartige Einrichtung oder Erhebung zwischen der Strahlungsquelle und dem Strahlungsdetektor ausgebildet. Bevorzugt besteht die Strahlungsbarriere z.B. aus Silizium. Diese Ausführungsform ist vorteilhaft, da die von der Strahlungsquelle emittierte Strahlung sehr gut vom Trommelfell und den dahinterliegenden Strukturen reflektiert wird, wodurch die Vitalparameter sehr genau erfasst werden können.

Der längliche Einbringanteil ist gemäß einer weiteren bevorzugten Ausführungsform der vorliegenden Erfindung dazu eingerichtet, die optische Sensoreinrichtung in einem Abstand von gleich oder weniger als 7mm zum Trommelfell, insbesondere zum Trommelfell eines definierten Gehörgangs, zu positionieren, insbesondere in einem Abstand zwischen 7mm und 3mm zum Trommelfell zu positionieren. Zusätzlich oder alternativ kann der längliche Einbringanteil eine Länge zwischen 12mm und 25mm, insbesondere zwischen 14mm und 23mm und besonders bevorzugt zwischen 16mm und 21mm und höchst bevorzugt von 18mm bzw. im Wesentlichen 18mm oder ca. 18mm oder genau 18mm, aufweisen. Beide Ausführungsformen bzw. Definitionen des Einbringanteil sind vorteilhaft, da dadurch der Abstand der optischen Sensoreinrichtung zum Trommelfell vorteilhaft vorgegeben wird.

Die Strahlungsquelle ist gemäß einer nicht beanspruchten Ausführungsform auf einer Oberseite eines Substrats zum Emittieren von Strahlung in Richtung einer Gehörgangwandung, insbesondere in radialer Richtung des Einbringanteils, angeordnet oder ausgebildet und der Strahlungsdetektor ist bevorzugt ebenfalls auf der Oberseite des Substrats zum Detektieren von Strahlung aus Richtung der Gehörgangwandung, insbesondere aus radialer Richtung des Einbringanteils, angeordnet oder ausgebildet. Die Strahlungsquelle und der Strahlungsdetektor sind bevorzugt in dieselbe Richtung orientiert, insbesondere derart, dass von der Strahlungsquelle emittierte Strahlung infolge von Reflexion durch den Strahlungsdetektor erfassbar ist. Das Substrat ist besonders bevorzugt eine Leiterplatine bzw. ein Printed Circuit Board (PCB), wobei das PCB bevorzugt mittels Leitungen, insbesondere flexiblen Leitungen, wie z.B. Kabeln, zum Übermitteln der erfassten Signale und/oder Daten mit dem Auswerteanteil verbindbar oder verbunden ist. Zwischen der Strahlungsquelle und dem Strahlungsdetektor ist bevorzugt eine Strahlungsbarriere ausgebildet. Besonders bevorzugt ist die Strahlungsbarriere als wandartige Einrichtung oder Erhebung zwischen der Strahlungsquelle und dem Strahlungsdetektor ausgebildet. Bevorzugt besteht die Strahlungsbarriere z.B. aus Silizium. Die Strahlungsquelle ist bevorzugt als zumindest zwei Leuchtmittel, insbesondere eine erste LED oder OLED und eine zweite LED oder OLED, zum Emittieren von Strahlung unterschiedlicher Wellenlängen ausgebildet. Diese Ausführungsform ist vorteilhaft, da die Strahlungsquelle und der Strahlungsdetektor sehr nah am Trommelfell in Richtung der Gehörgangswandung ausgerichtet sind, wodurch sehr präzise Vitalparameter erfassbar sind.

Der längliche Einbringanteil ist gemäß einer nicht beanspruchten Ausführungsform dazu eingerichtet, die optische Sensoreinrichtung in einem von Knochen umgebenen Anteil des Gehörgangs und/oder in einem Abstand von gleich oder weniger als 12mm zum Trommelfell, insbesondere zum Trommelfell eines definierten Gehörgangs, zu positionieren, insbesondere in einem Abstand zwischen 7mm und 3mm zum Trommelfell zu positionieren. Zusätzlich oder alternativ weist der längliche Einbringanteil eine Länge zwischen 12mm und 25mm, insbesondere zwischen 14mm und 23mm und besonders bevorzugt zwischen 16mm und 21mm und höchst bevorzugt von 18mm bzw. im Wesentlichen 18mm oder ca. 18mm oder genau 18mm, auf. Beide Ausführungsformen bzw. Definitionen des Einbringanteil sind vorteilhaft, da dadurch die Positionierung der optischen Sensoreinrichtung sehr nah zum Trommelfell beabstandet vorteilhaft vorgegeben wird.

Durch ein Leuchtmittel bzw. ein erstes Leuchtmittel der Strahlungsquelle ist gemäß einer weiteren bevorzugten Ausführungsform der vorliegenden Erfindung Strahlung mit Wellenlängen im Bereich von 860nm und 900nm, insbesondere im Bereich von 875nm und 885nm und bevorzugt von 880nm, emittierbar. Zusätzlich oder alternativ ist durch ein Leuchtmittel bzw. ein anderes Leuchtmittel oder ein weiteres Leuchtmittel der Strahlungsquelle gemäß einer weiteren bevorzugten Ausführungsform der vorliegenden Erfindung Strahlung mit Wellenlängen im Bereich von 620nm und 700nm, insbesondere im Bereich von 655nm und 665nm und bevorzugt von 660nm, emittierbar. Zusätzlich oder alternativ weist die Strahlungsquelle gemäß einer weiteren bevorzugten Ausführungsform der vorliegenden Erfindung ein Leuchtmittel bzw. ein weiteres Leuchtmittel bzw. ein drittes Leuchtmittel auf, wobei durch das dritte Leuchtmittel Strahlung im Bereich von 480nm und 580nm und bevorzugt in einem Bereich von 510nm und 545nm und besonders bevorzugt in einem Bereich von 520-535 nm emittierbar ist. Der Strahlungsdetektor ist besonders bevorzugt zum Detektieren von Strahlung des Leuchtmittels, des anderen Leuchtmittels und des dritten Leuchtmittels konfiguriert. Diese Ausführungsform ist vorteilhaft, da mittels der Strahlung des ersten Leuchtmittels und/oder der Strahlung des zweiten Leuchtmittels ein definierter Vitalparameter, insbesondere die Sauerstoffsättigung, erfassbar ist. Die Ausführungsform ist ferner vorteilhaft, da zusätzlich oder alternativ mittels dem Leuchtmittel bzw. dem dritten Leuchtmittel die Pulsrate erfassbar ist.

Die Strahlungsquelle und/oder der Strahlungsdetektor sind gemäß einer weiteren bevorzugten Ausführungsform der vorliegenden Erfindung von einem für die Strahlung der Strahlungsquelle transparentem und elastisch verformbaren Material, insbesondere einem Polymermaterial, umschlossen, insbesondere darin eingebettet. Diese Ausführungsform ist vorteilhaft, da durch das elastisch verformbare Material die sehr verletzlichen Bestandteile des Gehörgangs geschützt sind. Weiterhin wird durch das transparente Material ermöglich, dass die optische Erfassung des bzw. der Vitalparameter bewirkbar ist.

Das Substrat ist gemäß einer weiteren bevorzugten Ausführungsform der vorliegenden Erfindung mehrheitlich von einem elastisch verformbaren Material, insbesondere einem Polymermaterial, umschlossen, wobei das verformbare Material zumindest eine Ausnehmung ausbildet, wobei in der Ausnehmung die Strahlungsquelle und/oder der Strahlungsdetektor angeordnet sind. Die Ausführungsform ist vorteilhaft, da die Transparenzanforderung an das verformbare Material nicht gegeben ist, wodurch dieses kostengünstiger ausgewählt werden kann, insbesondere wenn das verformbare Material alternativ für mehrere Wellenlängenbereiche transparent sein müsste.

Eine Oberfläche der Strahlungsquelle, über welche die Strahlung der Strahlungsquelle emittierbar ist, ist gemäß einer weiteren bevorzugten Ausführungsform der vorliegenden Erfindung in einem ersten Abstand zum Substrat angeordnet, und wobei eine äußere Oberfläche des verformbaren Materials zumindest in dem die Ausnehmung umgebenen Bereich einen zweiten Abstand zum Substrat aufweist, wobei der zweite Abstand entweder gleich groß oder größer als der erste Abstand ist. Diese Ausführungsform ist vorteilhaft, da dadurch der Mindestabstand zwischen der Sensoreinrichtung und der Gehörgangsoberfläche definiert ist.

Der in den Gehörgang einbringbare längliche Einbringanteil bildet gemäß der vorliegenden Erfindung in seiner Längsrichtung ein inneres Ende aus und wobei der längliche Einbringanteil ein oder mindestens ein Abdichtungselement zum Verschließen des Gehörgangs aufweist oder ausbildet, wobei der Temperatursensor zwischen dem inneren Ende und dem Abdichtungselement angeordnet oder ausgebildet ist. Diese Ausführungsform ist vorteilhaft, da der Temperatursensor in einem Bereich anordenbar ist, in dem die Körperkerntemperatur erfassbar ist.

Die Anordnung des Temperatursensors zur Bestimmung der Körperkerntemperatur ist gemäß einer bevorzugten Ausführungsform der vorliegenden Erfindung derart konfiguriert, dass der Temperatursensor in einem Abstand von 9mm und 2mm oder in einem Abstand von weniger als 9mm, insbesondere weniger als 7mm oder zwischen 7mm und 2mm, und/oder in einem Abstand von mindestens 0,1mm vom Trommelfell beabstandet anordenbar ist, und wobei der Temperatursensor zur Bestimmung der Lufttemperatur eines zwischen dem Abdichtungselement und dem inneren Ende vorhandenen Luftvolumens eingerichtet ist. Diese Ausführungsform ist vorteilhaft, da durch das Abdichtungselement ein Bereich des Gehörgangs von der Umgebung abgegrenzt wird und sich in diesem Bereich die vorhandene Luft somit sehr schnell auf das Niveau der Körperkerntemperatur erwärmen lässt. Dadurch sind innerhalb kurzer Zeit sehr genaue Temperaturmessungen möglich.

Der Auswerteanteil und der Temperatursensor sind gemäß einer bevorzugten Ausführungsform der vorliegenden Erfindung zumindest zeitweise, insbesondere im gekoppelten Zustand, signaltechnisch miteinander verbunden sind. Der Temperatursensor ist bevorzugt ein von einem optischen Sensor verschiedener Sensor. Der Temperatursensor ist besonders bevorzugt ein Thermistor, wobei der Temperatursensor bevorzugt auf einer Rückseite des Substrats bzw. PCB angeordnet oder ausgebildet ist. Die Rückseite ist bevorzugt parallel oder bevorzugt abschnittsweise parallel zur Oberseite ausgebildet. Diese Lösung ist vorteilhaft, da mit einem sehr kleinen PCB mehrere Funktionsgruppen sehr nah am Trommelfell bzw. sehr tief im Gehörgang, insbesondere in inneren von Knochen umgebenen Gehörgangsanteil anordenbar sind.

Die oben genannte Aufgabe wird ebenfalls durch ein Verfahren gemäß Anspruch 10 zum Messen von Vitalparametern eines Menschen gelöst. Das erfindungsgemäße Verfahren weist bevorzugt mindestens die Schritte auf: Bereitstellen einer Vorrichtung zum Erfassen von zumindest zwei Vitalparametern, insbesondere eine erfindungsgemäße Vorrichtung, Einbringen des länglichen Einbringanteil in einem Gehörgang eines Menschen, Positionieren der optische Sensoreinrichtung in einem Abstand von 7mm oder von weniger als 7mm zum Trommelfell, Positionieren des Temperatursensors in einem Abstand von 16mm oder von weniger als 16mm zum Trommelfell, Abgrenzen eines Luft beinhaltenden Volumenanteils des Gehörgangs mittels eines Abdichtungselements des länglichen Einbringanteil gegenüber der Umgebung, Erzeugen von Temperaturdaten, insbesondere Kerntemperaturdaten, wobei die Temperaturdaten die Temperatur der im abgegrenzten Volumenanteil befindlichen Luft nach einer definierten Mindestdauer nachdem der Volumenanteil gegenüber der Umgebung abgegrenzt wurde repräsentieren, wobei die Temperaturdaten von dem Temperatursensor erzeugt werden oder aus analogen Sensorsignalen des Temperatursensors abgeleitet werden nachdem der Temperatursensor die Temperatur der im abgegrenzten Volumenanteil befindlichen Luft erfasst hat, Erzeugen von Vitalparameterdaten, wobei die Vitalparameterdaten von der optischen Sensoreinrichtung erzeugt werden oder aus analogen Sensorsignaldaten der optischen Sensoreinrichtung abgeleitet werden, wobei die Vitalparameterdaten zumindest einen Vitalparameter des Menschen repräsentieren, wobei die optische Sensoreinrichtung den zumindest einen Vitalparameter erfasst hat, Ansteuern einer Anzeigeeinrichtung in Abhängigkeit der Temperaturdaten und/oder der Vitalparameterdaten. Diese Lösung ist vorteilhaft, da durch die Anordnung der Sensoreinrichtung in unmittelbarer Nähe zum Trommelfell sehr präzise ein Vitalparameter oder eine Vielzahl an Vitalparametern erfasst werden kann.

Die optische Sensoreinrichtung wird gemäß einer weiteren bevorzugten Ausführungsform der vorliegenden Erfindung in einem Abstand von 6mm oder von weniger als 6mm zum Trommelfell positioniert, und der Temperatursensor wird bevorzugt in einem Abstand von 16mm oder von weniger als 16mm zum Trommelfell positioniert und der Auswerteanteil und der längliche Einbringanteil werden bevorzugt über eine Steckverbindung zur Erzeugung einer Datenverbindung, insbesondere Daten- und Energieverbindung, miteinander verbunden, wobei die optische Sensoreinrichtung die Vitalparameterdaten oder Vitalparametersignale an den Auswerteanteil übermittelt und wobei der Temperatursensor die Temperaturdaten oder Temperatursignale an den Auswerteanteil übermittelt. Zusätzlich oder alternativ kann die Vorrichtung zum Erfassen von zumindest zwei Vitalparametern, insbesondere der Sauerstoffsättigung und der Pulsfrequenz, mit einer Abdeckung zum vollständigen Abdecken des Ohrs und bevorzugt zur zumindest teilweisen und bevorzugt vollständigen Abdeckung des Mastoides verbunden werden, wobei die Abdeckung die Anzeigeeinrichtung, insbesondere ein Display, aufweist, wobei die Anzeigeeinrichtung in Abhängigkeit der Vitalparameterdaten und/oder Kerntemperaturdaten angesteuert wird. Zusätzlich oder alternativ kann der Auswerteanteil eine Kommunikationsschnittstelle zum kabellosen Datenaustausch aufweisen, wobei zwischen der Kommunikationsschnittstelle und einem mobilen Endgerät eine Datenverbindung zum Übermitteln der Vitalparameterdaten und/oder der Temperaturdaten oder zum Übermitteln von durch den Auswerteanteil erzeugten Auswertedaten aufgebaut wird, wobei die Auswertedaten aus den Vitalparameterdaten und/oder den Temperaturdaten erzeugt werden.

Einzelne oder alle Darstellungen der im Nachfolgenden beschriebenen Figuren sind bevorzugt als Konstruktionszeichnungen anzusehen, d.h. die sich aus der bzw. den Figuren ergebenden Abmessungen, Proportionen, Funktionszusammenhänge und/oder Anordnungen entsprechen bevorzugt genau oder bevorzugt im Wesentlichen denen der erfindungsgemäßen Vorrichtung/en bzw. des erfindungsgemäßen Produkts bzw. des erfindungsgemäßen Systems bzw. visualisieren im Wesentlichen oder genau Schritte des erfindungsgemäßen Verfahrens bzw. der erfindungsgemäßen Verfahren.

Darin zeigt:
- Fig. 1a: eine schematische Ansicht einer beispielhaften erfindungsgemäßen Vorrichtung zur Einbringung in einen Gehörgang eines Menschen;
- Fig. 1b: eine schematische und vergrößerte Ansicht der in Fig. 1a lediglich skizzierten Sensoreinrichtung;
- Fig. 1c: eine schematische Ansicht der beispielhaften erfindungsgemäßen Vorrichtung nach Fig. 1a eingesetzt in den Gehörgang eines Menschen;
- Fig. 2a: eine schematische Ansicht einer weiteren nicht beanspruchten Vorrichtung zum Einsetzen in den Gehörgang eines Menschen;
- Fig. 2b: eine schematische und vergrößerte Ansicht der in Fig. 2a lediglich skizzierten Sensoreinrichtung;
- Fig. 2c: eine schematische Ansicht der beispielhaften erfindungsgemäßen Vorrichtung nach Fig. 2a eingesetzt in den Gehörgang eines Menschen; und
- Fig. 3: eine schematische Ansicht eines alternativen Sensorkopfes einer nicht beanspruchten Vorrichtung.

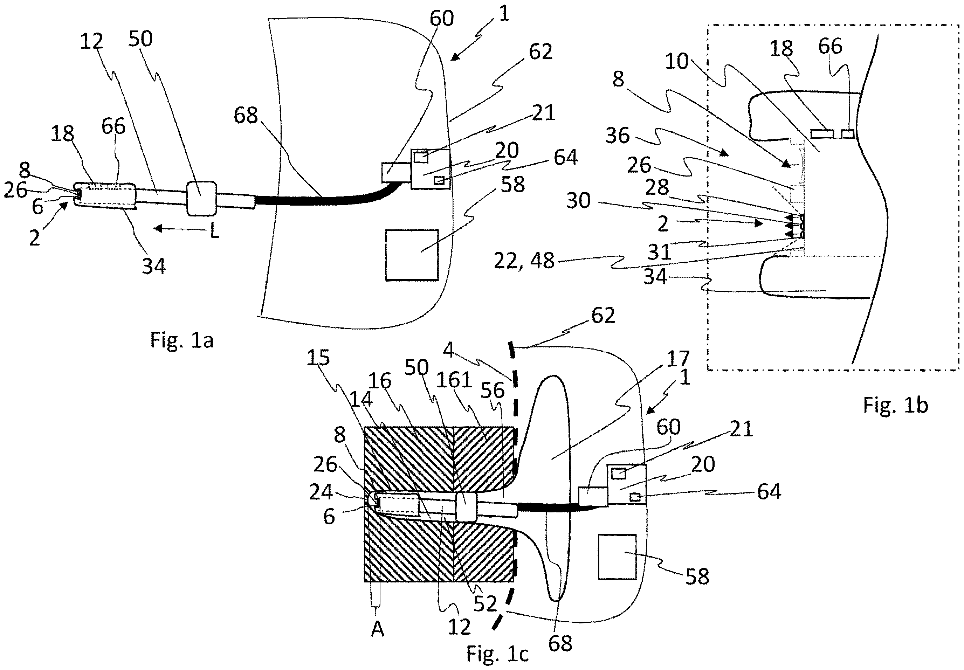

Fig. 1a zeigt ein Beispiel einer erfindungsgemäßen Vorrichtung 1. Die erfindungsgemäße Vorrichtung 1 ist dabei bevorzugt zum Erfassen eines oder mehrerer Vitalparameter ausgebildet. Als Vitalparameter wird hierbei beispielsweise die Körperkerntemperatur, die Sauerstoffsättigung des Blutes oder die Pulsfrequenz angesehen. Die Vorrichtung 1 kann somit alternativ als Vorrichtung zur Erfassung von Vitalparametern oder alternativ als Pulsfrequenzerfassungsvorrichtung oder als Sauerstoffsättigungserfassungsvorrichtung oder als Körperkerntemperaturerfassungsvorrichtung bezeichnet werden. Bevorzugt weist die Vorrichtung 1 mehrere miteinander lösbar koppelbare Anteile, nämlich einen länglichen Einbringanteil 12 und eine Abdeckung 62 auf. Der längliche Einbringanteil 12 ist dabei bevorzugt mittels einer Kopplungseinrichtung 60, insbesondere einer Steckverbindung, über welche besonders bevorzugt Signale und/oder Daten und/oder Strom übertragbar ist miteinander verbindbar. Es ist jedoch alternativ auch möglich, dass der längliche Einbringanteil 12 und die Abdeckung unlösbar miteinander verbunden sind.

Der längliche Einbringanteil 12 ist zur Einbringung in einen Gehörgang eines Lebewesens 4 (vgl. Fig. 1c), insbesondere Menschen, gestaltet und weist zumindest eine Sensoreinrichtung, insbesondere eine optische Sensoreinrichtung 2 und/oder einen Temperatursensor 18, auf. Die optische Sensoreinrichtung 2 ist bevorzugt auf einer Vorderseite 22 (vgl. Fig. 1b) des länglichen Einbringanteils 12 angeordnet oder ausgebildet. Die Vorderseite 22 ist bevorzugt im Wesentlichen orthogonal zur Längserstreckungsrichtung L des länglichen Einbringanteils 12 ausgerichtet. Bevorzugt bildet die Vorderseite 22 zumindest einen Teil oder mehrheitlich oder vollständig ein Ende 48 (vgl. Fig. 1b) des länglichen Einbringanteils 12 auf. Das von der Vorderseite 22 ausgebildete Ende kann auch als inneres Ende 48 bezeichnet werden, da es den in Längsrichtung L des länglichen Einbringanteil 12 am weitesten in den Gehörgang 14 des Ohrs 17 (vgl. Fig. 1c) des Lebewesens 4 (Fig. 1c) einbringbaren Anteil des länglichen Einbringanteils 12 ausbildet bzw. darstellt. Die optische Sensoreinrichtung 2 weist bevorzugt eine Strahlungsquelle 6 und einen Strahlungsdetektor 8 zum Detektieren von Strahlung 7, welche von der Strahlungsquelle 6 emittiert wurde auf. Besonders bevorzugt sind die Strahlungsquelle 6 und der Strahlungsdetektor 8 nebeneinander, insbesondere in einem Abstand von weniger als 5mm oder bevorzugt weniger als 4mm oder besonders bevorzugt weniger als 3mm zueinander angeordnet. Es ist hierbei möglich, dass eine Strahlungsbarriere 26 zwischen der Strahlungsquelle 6 und dem Strahlungsdetektor 8 vorgesehen bzw. ausgebildet ist. Die Strahlungsbarriere kann beispielsweise aus demselben Material bestehen, aus dem auch das Substrat 10 (vgl. Fig. 1b) besteht. Bevorzugt ist die Strahlungsbarriere 28 jedoch zumindest für Strahlung der Strahlungsquelle 6 nicht durchlässig.

Die optische Sensoreinrichtung 2 weist bevorzugt zumindest ein Leuchtmittel 28 und bevorzugt zwei oder mehr als zwei Leuchtmittel 28 und 30 und besonders bevorzugt drei oder mehr als drei Leuchtmittel 28, 30 und 31 (vgl. Fig. 1b) auf. Bevorzugt kann durch das erste Leuchtmittel 28 der Strahlungsquelle 6 Strahlung mit Wellenlängen im Bereich von 860nm und 900nm, insbesondere im Bereich von 875nm und 885nm und bevorzugt von 880nm, emittierbar sein. Ein anderes bzw. zweites Leuchtmittel 30 der Strahlungsquelle 6 ist bevorzugt zum emittieren von Strahlung mit Wellenlängen im Bereich von 620nm und 700nm, insbesondere im Bereich von 655nm und 665nm und bevorzugt von 660nm, ausgebildet. Bevorzugt kann die Strahlungsquelle 6 zumindest ein drittes Leuchtmittel 31 aufweisen, wobei durch das dritte Leuchtmittel 31 Strahlung im Bereich von 480nm und 580nm und bevorzugt in einem Bereich von 510nm und 545nm und besonders bevorzugt in einem Bereich von 520-535 nm emittierbar ist. Es ist hierbei selbstverständlich möglich, dass -je nach erforderlichen Vitalparametern - das als drittes Leuchtmittel 31 bezeichnete Leuchtmittel als erstes Leuchtmittel oder als zweites Leuchtmittel verstanden wird. Ebenso kann für das zweite Leuchtmittel 30 gelten, dass es alternativ als erstes oder drittes Leuchtmittel verstanden werden kann. Das erste Leuchtmittel 29 kann somit alternativ als zweites oder drittes Leuchtmittel verstanden werden.

Der Strahlungsdetektor 8 zum Detektieren von Strahlung 7 der Strahlungsquelle 6 ist somit konfiguriert Strahlung des ersten Leuchtmittels 28 und des zweiten Leuchtmittels 30 und des dritten Leuchtmittels 31 zu erfassen und in Daten oder analoge Signale zu überführen.

Der Temperatursensor 18 ist bevorzugt auf einer zur Vorderseite 22 (vgl. Fig. 1b) geneigten Oberfläche des länglichen Einbringanteils 12 angeordnet oder ausgebildet. Weiterhin kann ein Kondensator 66, insbesondere zur Versorgung der Strahlungsquelle 6 und/oder des Strahlungsdetektors 8 und/oder des Temperatursensors 18 mit elektrischer Energie, auf dem Substrat angeordnet oder ausgebildet sein.

Ferner weist der längliche Einbringanteil 12 bevorzugt ein Abdichtungselement 50 zum Abdichten eines Gehörgangs 14 (vgl. Fig. 1c) auf. Das Abdichtungselement 50 ist dabei bevorzugt dauerhaft oder austauschbar am länglichen Einbringanteil 12 angeordnet. Besonders bevorzugt lässt sich die Position des Abdichtungselements 50 in Längsrichtung L des längliche Einbringanteils 12 einstellen oder verschieben. Alternativ ist es jedoch auch möglich, dass das Abdichtungselement 50 an einer definierten Position fest (jedoch bevorzugt austauschbar) angeordnet ist.

Das Bezugszeichen 68 kennzeichnet ein Verbindungselement, welches bevorzugt mittels einer Kopplungseinrichtung 60, insbesondere einer lösbaren Steckverbindung oder einer lösbaren magnetischen Verbindung, mit dem Auswerteanteil 20, welcher bevorzugt über zumindest einen Microprozessor 21 zum Aufbereiten der Vitalparameterdaten und/oder zum Ansteuern der Sensoreinrichtung 2 und/oder dem Temperatursensor 18 verfügt, verbunden ist.

Signale und/oder Daten und/oder elektrische Energie ist dabei mittels einem oder mehreren Leitungselement/en (nicht gezeigt) zwischen der Sensoreinrichtung 2 und/oder dem Temperatursensor 18 und dem Auswertanteil 20 leitbar. Das Leitungselement oder die Leitungselemente verlaufen dabei bevorzugt im Inneren des länglichen Einbringanteils 20 sowie im Inneren des Verbindungselements 68. Der längliche Einbringanteil 20 und das Verbindungselement 68 können alternativ als eine kontinuierliche Einrichtung bzw. als Einrichtung mit einem gemeinsamen Gehäuse oder Einhausung, insbesondere als länglicher Einbringanteil 20 mit äußerem Verbindungsabschnitt, ausgeführt sein. Das Bezugszeichen 58 kennzeichnet eine Anzeigeeinrichtung, insbesondere zum Anzeigen von Informationen, insbesondere Vitalparameterergebnisse oder Veränderungen von Vitalparametern oder Bedienanweisungen. Weiterhin können auch Eingabemittel (nicht gezeigt), insbesondere Taster und Schalter, zum Eingeben von Steuerbefehlen vorgesehen sein. Bevorzugt ist die Anzeigeeinrichtung 58 als Touchscreen ausgebildet und ermöglich somit neben der optischen Ausgabe von Information auch die Auswahl oder Ansteuerung von Funktionen. Ferner kann die Vorrichtung 1 eine akustische Ausgabeeinrichtung (nicht gezeigt) zur akustischen Ausgabe von Informationen, insbesondere Vitalparameterergebnisse oder Veränderungen von Vitalparametern oder Bedienanweisungen, aufweisen. Weiterhin weist der Auswerteanteil 20 bevorzugt ein Kommunikationsmittel bzw. eine Kommunikationseinrichtung 64, insbesondere eine Kommunikationseinrichtung zum kabellosen Datenaustausch, insbesondere zum Datenaustausch per Funkübertragung, wie z.B. NFC oder Bluetooth oder WLAN oder 3G, 4G, 5G oder 6G, auf oder ist mit einer solchen Kommunikationseinrichtung 64 verbunden.

Fig. 1b zeigt eine vergrößerte Darstellung des im Bereich des Trommelfells 24 (vgl. Fig. 1c) anordenbaren Anteils der Vorrichtung 1. Es ist erkennbar, dass das elastisch verformbare Material 34 bevorzugt eine Ausnehmung aufweist oder ausbildet, wobei die Strahlung 7, welche von den Leuchtmitteln 28, 30, 31 emittiert wird, über das Trommelfell 24 in das Lebewesen 4 einleitbar ist und reflektierte Strahlung 7 zum Strahlungsdetektor 8 gelangen kann, ohne dass die Strahlung durch nichtbiologisches Material hindurchdringen muss. Das Bezugszeichen 36 kennzeichnet hierbei eine Ausnehmung, d.h. einen nicht vom elastischen Material 34 gefüllten Bereich im Strahlengang der Strahlung 7. Alternativ kann das elastisch verformbare Material 34 die Sensoreinrichtung 2 vollständig einhausen, wobei in diesem Fall das elastisch verformbare Material für die von dem mindestens einen Leuchtmittel und bevorzugt von den Leuchtmitteln emittierte Strahlung 7 transparent sein müsste.

Fig. 1c zeigt schematisch eine erfindungsgemäße Vorrichtung 1, wobei der längliche Einbringanteil 12 in den Gehörgang 14 eines Lebewesens 4 eingebracht ist. Der elastisch verformbare Anteil 34 kontaktiert dabei die Gehörgangswandung 15 und verhindert z.B., dass scharfkantige Anteile des länglichen Anteils 12 mit der sehr empfindlichen Gehörgangswandung 15 in Kontakt kommt.

Die Sensoreinrichtung 2 ist in der gezeigten Anordnung in einem Abstand A von weniger als 7mm zum Trommelfell 24 beabstandet angeordnet.

Das Abdichtungselement 50 ist bevorzugt derart angeordnet, dass in einem von Gewebe umgebenen Anteil 161 des Gehörgangs 14 positioniert wird. Alternativ kann das Abdichtelement 50 jedoch auch derart angeordnet sein, dass es in einem von Knochen umgebenen Anteil 16 des Gehörgangs 14 positioniert wird. Infolge der Positionierung des Abdichtungselements 50 wird ein Luftvolumen 52, welches sich zwischen dem Abdichtungselement 50 und dem Trommelfell 24 befindet von der Umgebung 56 abgegrenzt.

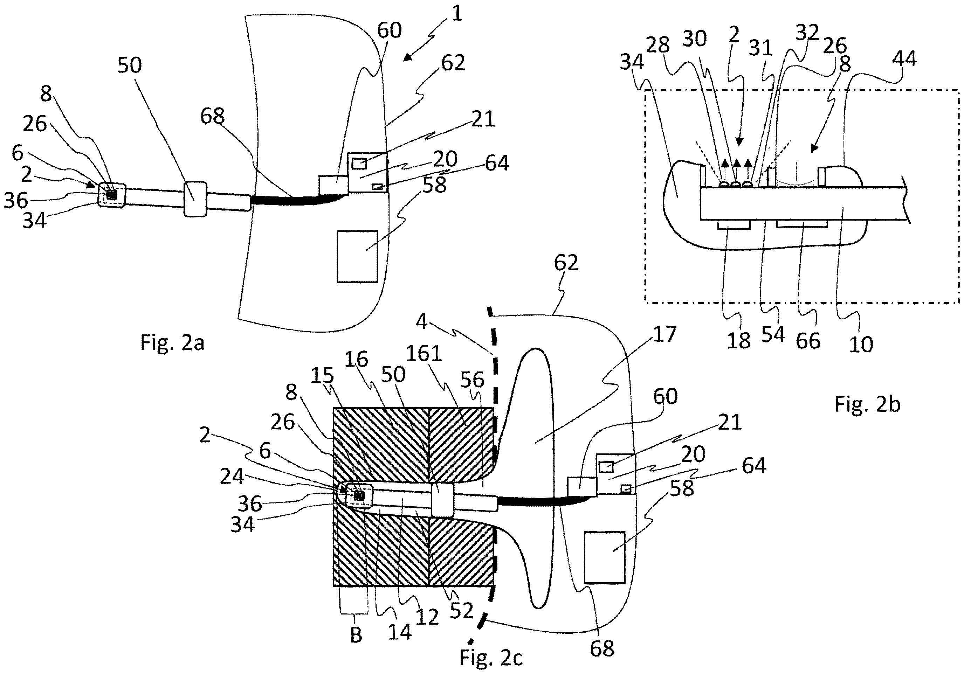

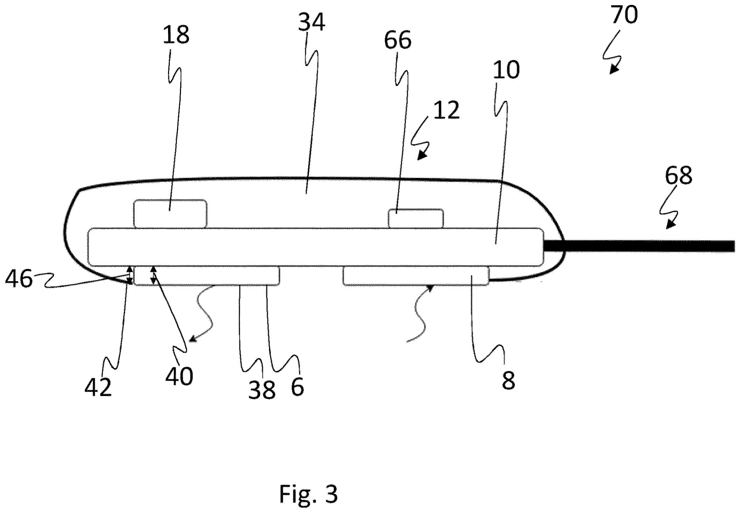

Die Fig. 2a-2c zeigen ein nicht beanspruchtes Beispiel der Vorrichtung 1. Die in den Fig. 2a-2c gezeigte Vorrichtung 1 stimmt im Wesentlichen mit der in Fig. 1a-1c gezeigten Vorrichtung 1 überein, wobei lediglich die Anordnung der Sensoreinrichtung 2 und/oder des Temperatursensors 18 verschieden ist, wodurch für dieselben Komponenten dieselben Bezugszeichen verwendet werden und die zuvor genannten Erläuterungen analog gelten. Es ist hierbei z.B. Fig. 2b entnehmbar, dass die Strahlungsquelle 6 auf einer Oberseite 32 eines Substrats 10 zum Emittieren von Strahlung 7 in Richtung einer Gehörgangwandung 15 (vgl. Fig. 2c), insbesondere in radialer Richtung des Einbringanteils 12, angeordnet oder ausgebildet ist. Der Strahlungsdetektor 8 ist ebenfalls auf der Oberseite 32 des Substrats 10 zum Detektieren von Strahlung 7 aus Richtung der Gehörgangwandung 15 (vgl. Fig. 2c), insbesondere aus radialer Richtung des Einbringanteils 12, angeordnet oder ausgebildet. Das Substrat 10 ist bevorzugt als ein PCB ausgeführt und zwischen der Strahlungsquelle 6 und dem Strahlungsdetektor 8 ist besonders bevorzugt eine Strahlungsbarriere 26 ausgebildet ist. Die Strahlungsquelle 6 kann dabei als zumindest zwei Leuchtmittel 28, 30, insbesondere eine erste LED oder OLED und eine zweite LED oder OLED, zum Emittieren von Strahlung 7 unterschiedlicher Wellenlängen ausgebildet sein. Der längliche Einbringanteil 12 ist bevorzugt dazu eingerichtet, dass die optische Sensoreinrichtung 2 in einem von Knochen umgebenen Anteil 16 des Gehörgangs 14 und/oder in einem Abstand (B) von gleich oder weniger als 12mm zum Trommelfell 24 (vgl. Fig. 2c), insbesondere zum Trommelfell 24 eines definierten Gehörgangs 14, positionierbar ist, insbesondere in einem Abstand zwischen 7mm und 3mm zum Trommelfell 24 (vgl. Fig. 2c) positionierbar ist. Fig. 3 zeigt ein weiteres Beispiel eines Sensorkopfes 70. Der Sensorkopf 70 wird dabei durch den länglichen Einbringanteil 12 und zumindest einen Anteil des Verbindungselements 68 ausgebildet. Das Bezugszeichen 38 kennzeichnet dabei eine vom Substrat 10 beabstandete Oberfläche, über welche die Strahlung 7 emittierbar ist. Die Oberfläche 38 ist dabei in einem Abstand 40 zum nächstliegenden Substratoberflächenanteil beabstandet. Das Bezugszeichen 34 kennzeichnet ein elastisches Material, welches zum Schutz des Gehörgangs 14 (vgl. Fig. 1c oder Fig. 2c) vorgesehen ist. Das elastische Material hat in einem an die Strahlungsquelle 6, insbesondere unmittelbar, angrenzenden Bereich einen Abstand 42 zum nächstliegenden Substratoberflächenanteil. Der Abstand 42 ist bevorzugt gleich dem Abstand 40 oder der Abstand 42 ist größer als der Abstand 40.

Die vorliegende Offenbarung bezieht sich somit allgemein auf eine Vorrichtung 1 zum Erfassen von zumindest zwei Vitalparametern, wobei diese Vorrichtung 1 eine optische Sensoreinrichtung 2, einen länglichen Einbringanteil 12 und einen Auswerteanteil 20 aufweist. Die optische Sensoreinrichtung 2 dient bevorzugt zur Erfassung von mindestens einem Vitalparameter eines Lebewesens 4, insbesondere Menschen, wobei die optische Sensoreinrichtung 2 eine Strahlungsquelle 6 zum Emittieren von Strahlung 7 und einen Strahlungsdetektor 8 zum Detektieren von Strahlung 7 aufweist, wobei die Strahlungsquelle 6 und der Strahlungsdetektor 8 besonders bevorzugt nebeneinander angeordnet sind. Der längliche Einbringanteil 12 dient bevorzugt zum Einbringen in einen Gehörgang 14 und zum Positionieren der optischen Sensoreinrichtung 2 in einem von Knochen umgebenen Anteil 16 des Gehörgangs 14. Die optische Sensoreinrichtung 2 ist bevorzugt an dem länglichen Einbringanteil 12 angeordnet oder ist Bestandteil davon. Ein Temperatursensor 18 zur Erfassung der Körperkerntemperatur ist bevorzugt an dem länglichen Einbringanteil 12 angeordnet oder Bestandteil davon. Der Auswerteanteil 20 ist bevorzugt mit dem Einbringanteil 12 gekoppelt, insbesondere lösbar gekoppelt, wobei der Auswerteanteil 20 und die optische Sensoreinrichtung 2 zumindest zeitweise, insbesondere in einem gekoppelten Zustand, signaltechnisch miteinander verbunden sind. Weiterhin lässt sich Fig. 2b entnehmen, dass der Temperatursensor 18 und/oder der Kondensator 66 auf einer der Oberseite 32 abgewandten Rückseite 54 angeordnet oder ausgebildet ist/sind. Es ist hierbei jedoch alternativ möglich, dass der Temperatursensor 18 und/oder der Kondensator 66 auf derselben Seite, d.h. z.B. auf der Oberseite 32, angeordnet oder ausgebildet ist auf der ebenfalls die Sensoreinrichtung 2 angeordnet oder ausgebildet ist.

### Bezugszeichenliste

- 1: Vorrichtung zur Erfassung von zumindest einem Vitalparameter und bevorzugt zumindest zwei Vitalparametern
- 2: optische Sensoreinrichtung
- 4: Lebewesen
- 6: Strahlungsquelle
- 7: Strahlung
- 8: Strahlungsdetektor
- 10: Substrat
- 12: länglicher Einbringanteil
- 14: Gehörgang
- 15: Gehörgangswandung
- 16: von Knochen umgebener Anteil
- 17: Ohr
- 18: Temperatursensor
- 20: Auswerteanteil
- 22: Vorderseite
- 24: Trommelfell
- 26: Strahlungsbarriere
- 28: erstes Leuchtmittel
- 30: zweites Leuchtmittel
- 31: drittes Leuchtmittel
- 32: Oberseite
- 34: elastisch verformbares Material
- 36: Ausnehmung
- 38: Oberfläche
- 40: erster Abstand
- 42: äußere Oberfläche
- 44: umgebende Bereich
- 46: zweiter Abstand
- 48: inneres Ende
- 50: Abdichtungselement
- 52: Luftvolumen
- 54: Rückseite
- 56: Umgebung
- 58: Anzeigeeinrichtung
- 60: Steckverbindung
- 62: Abdeckung
- 64: Kommunikationsschnittstelle
- 66: Kondensator
- 68: Verbindungselement
- 70: Sensorkopf
- 161: von Gewebe umgebener Anteil
- A: Abstand zwischen Trommelfell und auf Vorderseite angeordneter Sensoreinrichtung
- B: Abstand zwischen Trommelfell und auf Oberseite angeordneter Sensoreinrichtung
- L: Längserstreckungsrichtung des länglichen Einbringanteils

## Patentansprüche

1. Vorrichtung (1) zum Erfassen von zumindest zwei Vitalparametern,
mindestens aufweisend
eine optische Sensoreinrichtung (2) zur Erfassung von mindestens einem Vitalparameter
eines Lebewesens (4), wobei die optische Sensoreinrichtung (2) eine Strahlungsquelle (6) zum Emittieren von Strahlung (7) und einen Strahlungsdetektor (8) zum Detektieren von Strahlung (7) aufweist, wobei die Strahlungsquelle (6) und der Strahlungsdetektor (8) nebeneinander angeordnet sind,
einen länglichen Einbringanteil (12) zum Einbringen in einen Gehörgang (14) und zum Positionieren der optischen Sensoreinrichtung (2) in einem von Knochen umgebenen Anteil (16) des Gehörgangs (14),
wobei die optische Sensoreinrichtung (2) an dem länglichen Einbringanteil (12) angeordnet ist oder Bestandteil davon ist und wobei ein Temperatursensor (18) zur Erfassung der Körperkerntemperatur an dem länglichen Einbringanteil (12) angeordnet ist oder Bestandteil davon ist,
ein mit dem Einbringanteil (12) gekoppelter, insbesondere lösbar gekoppelter, Auswerteanteil (20), wobei der Auswerteanteil (20) und die optische Sensoreinrichtung (2) zumindest zeitweise, insbesondere in einem gekoppelten Zustand, signaltechnisch miteinander verbunden sind,
wobei der in den Gehörgang (14) einbringbare längliche Einbringanteil (12) in seiner Längsrichtung (L) ein inneres Ende (48) ausbildet und wobei der längliche Einbringanteil (12) ein oder mindestens ein Abdichtungselement (50) zum Verschließen des Gehörgangs (14) aufweist oder ausbildet, wobei der Temperatursensor (18) zwischen dem inneren Ende (48) und dem Abdichtungselement (50) angeordnet oder ausgebildet ist,
**dadurch gekennzeichnet, dass**
die Strahlungsquelle (6) auf einer Vorderseite (22) eines Substrats (10) zum Emittieren von Strahlung (7) in Richtung eines Trommelfells (24) in Verlängerungsrichtung des Einbringanteils (12) angeordnet oder ausgebildet ist und
der Strahlungsdetektor (8) auf der Vorderseite (22) des Substrats (10) zum Detektieren von Strahlung (7) aus Richtung des Trommelfells (24)in der Verlängerungsrichtung des Einbringanteils (12) angeordnet oder ausgebildet ist,
wobei das Substrat (10) ein PCB ist und
wobei zwischen der Strahlungsquelle (6) und dem Strahlungsdetektor (8) eine Strahlungsbarriere (26) ausgebildet ist,
wobei die Strahlungsquelle (6) als zumindest zwei Leuchtmittel (28, 30), insbesondere eine erste LED oder OLED und eine zweite LED oder OLED, zum Emittieren von Strahlung (7) unterschiedlicher Wellenlängen ausgebildet ist.

2. Vorrichtung nach Anspruch 1,
**dadurch gekennzeichnet, dass**
der längliche Einbringanteil (12) dazu eingerichtet ist, die optische Sensoreinrichtung (2) in einem Abstand von gleich oder weniger als 7mm zum Trommelfell (24), insbesondere zum Trommelfell (24) eines definierten Gehörgangs (14), zu positionieren, insbesondere in einem Abstand zwischen 7mm und 3mm zum Trommelfell (24) zu positionieren und/oder
der längliche Einbringanteil (12) eine Länge zwischen 12mm und 25mm aufweist.

3. Vorrichtung nach einem der Ansprüche 1 bis 2,
**dadurch gekennzeichnet, dass**
durch ein Leuchtmittel (28) der Strahlungsquelle (6) Strahlung mit Wellenlängen im Bereich von 860nm und 900nm, insbesondere im Bereich von 875nm und 885nm und bevorzugt von 880nm, emittierbar ist
und
wobei durch ein anderes Leuchtmittel (30) der Strahlungsquelle (6) Strahlung mit Wellenlängen im Bereich von 620nm und 700nm, insbesondere im Bereich von 655nm und 665nm und bevorzugt von 660nm, emittierbar ist.

4. Vorrichtung nach Anspruch 3,
**dadurch gekennzeichnet, dass**
die Strahlungsquelle (6) zumindest ein drittes Leuchtmittel (31) aufweist, wobei durch das dritte Leuchtmittel (31) Strahlung im Bereich von 480nm und 580nm und bevorzugt in einem Bereich von 510nm und 545nm und besonders bevorzugt in einem Bereich von 520-535 nm emittierbar ist,
wobei der Strahlungsdetektor (8) zum Detektieren von Strahlung (7) des Leuchtmittels (28), des anderen Leuchtmittels (30) und des dritten Leuchtmittels (31) konfiguriert ist.

5. Vorrichtung nach einem der Ansprüche 1 bis 4,
**dadurch gekennzeichnet, dass**
die Strahlungsquelle (6) und/oder der Strahlungsdetektor (8) von einem für die Strahlung (7) der Strahlungsquelle (6) transparentem und elastisch verformbaren Material (34) umschlossen, insbesondere darin eingebettet, sind.

6. Vorrichtung nach einem der Ansprüche 1 bis 5,
**dadurch gekennzeichnet, dass**
das Substrat (10) mehrheitlich von einem elastisch verformbaren Material (34) umschlossen ist, wobei das elastisch verformbare Material (34) zumindest eine Ausnehmung (36) ausbildet, wobei in der Ausnehmung (36) die Strahlungsquelle (6) und/oder der Strahlungsdetektor (8) angeordnet sind.

7. Vorrichtung nach Anspruch 6,
**dadurch gekennzeichnet, dass**
eine Oberfläche (38) der Strahlungsquelle (6), über welche die Strahlung (7) der Strahlungsquelle (6) emittierbar ist, in einem ersten Abstand (40) zum Substrat (10) angeordnet ist, und wobei eine äußere Oberfläche (42) des elastisch verformbaren Materials (34) zumindest in dem die Ausnehmung (36) umgebenen Bereich (44) einen zweiten Abstand (46) zum Substrat (10) aufweist, wobei der zweite Abstand (46) entweder gleich groß oder größer als der erste Abstand (40) ist.

8. Vorrichtung nach Anspruch 1,
**dadurch gekennzeichnet, dass**
die Anordnung des Temperatursensors (18) zur Bestimmung der Körperkerntemperatur derart konfiguriert ist, dass der Temperatursensor (18) in einem Abstand von 9mm und 2mm oder in einem Abstand von weniger als 9mm, insbesondere weniger als 7mm oder zwischen 7mm und 2mm, und/oder in einem Abstand von mindestens 0,1mm vom Trommelfell (18) beabstandet anordenbar ist, und wobei der Temperatursensor (18) zur Bestimmung der Lufttemperatur eines zwischen dem Abdichtungselement (50) und dem inneren Ende (48) vorhandenen Luftvolumens (52) eingerichtet ist.

9. Vorrichtung nach Anspruch 8,
**dadurch gekennzeichnet, dass**
der Auswerteanteil (20) und der Temperatursensor (18) zumindest zeitweise, insbesondere im gekoppelten Zustand, signaltechnisch miteinander verbunden sind, wobei der Temperatursensor (18) ein von einem optischen Sensor verschiedener Sensor ist, wobei der Temperatursensor (18) ein Thermistor ist, wobei der Temperatursensor bevorzugt auf einer Rückseite (54) des Substrats (10) angeordnet oder ausgebildet ist, wobei die Rückseite (54) bevorzugt parallel oder bevorzugt abschnittsweise parallel zur Oberseite (32) ausgebildet ist.

10. Verfahren zum Messen von Vitalparametern eines Menschen (4), mindestens umfassend die Schritte:
Bereitstellen einer Vorrichtung (1) zum Erfassen von zumindest zwei Vitalparametern nach einem der vorangegangenen Ansprüche,
Einbringen des länglichen Einbringanteil (12) in einem Gehörgang (14) eines Menschen (4), Positionieren der optische Sensoreinrichtung (2) in einem Abstand von 7mm oder von weniger als 7mm zum Trommelfell (24),
Positionieren des Temperatursensors (18) in einem Abstand von 16mm oder von weniger als 16mm zum Trommelfell (24),
Abgrenzen eines Luft beinhaltenden Volumenanteils (52) des Gehörgangs (14) mittels eines Abdichtungselements (50) des länglichen Einbringanteil (12) gegenüber der Umgebung (56), Erzeugen von Temperaturdaten, insbesondere Kerntemperaturdaten, wobei die Temperaturdaten die Temperatur der im abgegrenzten Volumenanteil (52) befindlichen Luft nach einer definierten Mindestdauer nachdem der Volumenanteil (52) gegenüber der Umgebung (56) abgegrenzt wurde repräsentieren, wobei die Temperaturdaten von dem Temperatursensor (18) erzeugt werden oder aus analogen Sensorsignalen des Temperatursensors (18) abgeleitet werden nachdem der Temperatursensor (18) die Temperatur der im abgegrenzten Volumenanteil befindlichen Luft erfasst hat,
Erzeugen von Vitalparameterdaten, wobei die Vitalparameterdaten von der optischen Sensoreinrichtung (2) erzeugt werden oder aus analogen Sensorsignaldaten der optischen Sensoreinrichtung (2) abgeleitet werden, wobei die Vitalparameterdaten zumindest einen Vitalparameter des Menschen (4) repräsentieren, wobei die optische Sensoreinrichtung (2) den zumindest einen Vitalparameter erfasst hat,
Ansteuern einer Anzeigeeinrichtung (58) in Abhängigkeit der Temperaturdaten und/oder der Vitalparameterdaten.

11. Verfahren nach Anspruch 10,
**dadurch gekennzeichnet, dass**
die optische Sensoreinrichtung (2) in einem Abstand von 6mm oder von weniger als 6mm zum Trommelfell (24) positioniert wird, und
der Temperatursensor (18) in einem Abstand von 16mm oder von weniger als 16mm zum Trommelfell (24) positioniert wird
und
wobei der Auswerteanteil (20) und der längliche Einbringanteil (12) über eine Steckverbindung (60) zur Erzeugung einer Datenverbindung, insbesondere Daten- und Energieverbindung, miteinander verbunden werden, wobei die optische Sensoreinrichtung (2) die Vitalparameterdaten oder Vitalparametersignale an den Auswerteanteil (20) übermittelt und wobei der Temperatursensor (18) die Temperaturdaten oder Temperatursignale an den Auswerteanteil (20) übermittelt
und
wobei die Vorrichtung (1) zum Erfassen von zumindest zwei Vitalparametern, insbesondere der Sauerstoffsättigung und der Pulsfrequenz, mit einer Abdeckung (62) zum vollständigen Abdecken des Ohrs (17) und bevorzugt zur zumindest teilweisen und bevorzugt vollständigen Abdeckung des Mastoides verbunden wird, wobei die Abdeckung (62) die Anzeigeeinrichtung (58), insbesondere ein Display (58), aufweist, wobei die Anzeigeeinrichtung (58) in Abhängigkeit der Vitalparameterdaten und/oder Kerntemperaturdaten angesteuert wird
und
wobei der Auswerteanteil (20) eine Kommunikationsschnittstelle (64) zum kabellosen Datenaustausch aufweist, wobei zwischen der Kommunikationsschnittstelle (64) und einem mobilen Endgerät (66) eine Datenverbindung (68) zum Übermitteln der Vitalparameterdaten und/oder der Temperaturdaten oder zum Übermitteln von durch den Auswerteanteil (20) erzeugten Auswertedaten aufgebaut wird, wobei die Auswertedaten aus den Vitalparameterdaten und/oder den Temperaturdaten erzeugt werden.

## Claims

1. Device (1) for detecting at least two vital parameters,
comprising at least
an optical sensor device (2) for capturing at least one vital parameter of a living being (4), wherein the optical sensor device (2) comprises a radiation source (6) for emitting radiation (7) and a radiation detector (8) for detecting radiation (7), wherein the radiation source (6) and the radiation detector (8) are arranged next to each other,
a longitudinal insertion portion (12) for insertion into an ear canal (14) and for positioning the optical sensor device (2) in a portion (16) of the ear canal (14) surrounded by bone,
wherein the optical sensor device (2) is arranged at the longitudinal insertion portion (12) or is a component thereof and wherein a temperature sensor (18) for capturing the body core temperature is arranged at the longitudinal insertion portion (12) or is a component thereof,
an evaluation portion (20) coupled, in particular detachably coupled, to the insertion portion (12), wherein the evaluation portion (20) and the optical sensor device (2) are at least temporarily, in particular in a coupled state, signal-technically connected to one another,
wherein the longitudinal insertion portion (12), which can be inserted into the ear canal (14), forms an inner end (48) in its longitudinal direction (L) and wherein the longitudinal insertion portion (12) comprises or forms one or at least one sealing element (50) for closing the ear canal (14), wherein the temperature sensor (18) is arranged or formed between the inner end (48) and the sealing element (50),
**characterized in that**
the radiation source (6) is arranged or formed on a front side (22) of a substrate (10) for emitting radiation (7) in the direction of a tympanic membrane (24) in the extension direction of the insertion portion (12), and
the radiation detector (8) is arranged or formed on the front side (22) of the substrate (10) for detecting radiation (7) from the direction of the tympanic membrane (24) in the extension direction of the insertion portion (12),
wherein the substrate (10) is a PCB and
wherein a radiation barrier (26) is formed between the radiation source (6) and the radiation detector (8),
wherein the radiation source (6) is formed as at least two light sources (28, 30), in particular a first LED or OLED and a second LED or OLED, for emitting radiation (7) of different wavelengths.

2. Device according to claim 1,
**characterized in that**
the longitudinal insertion portion (12) is arranged to position the optical sensor device (2) at a distance of equal to or less than 7 mm from the tympanic membrane (24), in particular from the tympanic membrane (24) of a defined ear canal (14), in particular at a distance of between 7 mm and 3 mm from the tympanic membrane (24)
and/or
the longitudinal insertion portion (12) comprises a length between 12mm and 25mm.

3. Device according to one of claims 1 to 2,
**characterized in that**
radiation with wavelengths in the range of 860nm and 900nm, in particular in the range of 875nm and 885nm and preferably 880nm, can be emitted by a light source (28) of the radiation source (6) and
wherein radiation with wavelengths in the range of 620nm and 700nm, in particular in the range of 655nm and 665nm and preferably of 660nm, can be emitted by another light source (30) of the radiation source (6).

4. Device according to claim 3,
**characterized in that**
the radiation source (6) comprises at least one third light source (31), wherein radiation in the range of 480 nm and 580 nm and preferably in a range of 510 nm and 545 nm and particularly preferably in a range of 520-535 nm can be emitted by the third light source (31),
wherein the radiation detector (8) is configured to detect radiation (7) from the light source (28), the other light source (30) and the third light source (31).

5. Device according to any one of claims 1 to 4,
**characterized in that**
the radiation source (6) and/or the radiation detector (8) are enclosed by a material (34) transparent to the radiation (7) of the radiation source (6) and elastically deformable, in particular embedded therein.

6. Device according to one of claims 1 to 5,
**characterized in that**
the substrate (10) is mainly enclosed by an elastically deformable material (34), wherein the elastically deformable material (34) forms at least one recess (36), the radiation source (6) and/or the radiation detector (8) being arranged in the recess (36).

7. Device according to claim 6,
**characterized in that**
a surface (38) of the radiation source (6), via which the radiation (7) of the radiation source (6) can be emitted, is arranged at a first distance (40) from the substrate (10), and wherein an outer surface (42) of the elastically deformable material (34) comprises a second distance (46) from the substrate (10) at least in the region (44) surrounding the recess (36), wherein the second distance (46) is either equal to or greater than the first distance (40).

8. Device according to claim 1,
**characterized in that**
the arrangement of the temperature sensor (18) for determining the body core temperature is configured such that the temperature sensor (18) can be arranged at a distance of 9mm and 2mm or at a distance of less than 9mm, in particular less than 7mm or between 7mm and 2mm, and/or at a distance of at least 0.1mm from the tympanic membrane (18), and wherein the temperature sensor (18) is set up to determine the air temperature of an air volume (52) present between the sealing element (50) and the inner end (48).

9. Device according to claim 8,
**characterized in that**
the evaluation portion (20) and the temperature sensor (18) are at least temporarily, in particular in the coupled state, connected to one another by signal technology, wherein the temperature sensor (18) is a sensor different from an optical sensor, wherein the temperature sensor (18) is a thermistor, wherein the temperature sensor is preferably arranged or formed on a rear side (54) of the substrate (10), wherein the rear side (54) is preferably formed parallel or preferably in sections parallel to the upper side (32).

10. Method for measuring vital parameters of a human (4),
comprising at least the steps of:
Providing a device (1) for capturing at least two vital parameters according to one of the preceding claims,
inserting the longitudinal insertion portion (12) into an ear canal (14) of a human (4),
positioning the optical sensor device (2) at a distance of 7 mm or less than 7 mm from the tympanic membrane (24),
positioning the temperature sensor (18) at a distance of 16mm or less than 16mm from the tympanic membrane (24),
delimiting an air-containing volume portion (52) of the ear canal (14) by means of a sealing element (50) of the longitudinal insertion portion (12) with respect to the environment (56),
generating temperature data, in particular core temperature data, wherein the temperature data represent the temperature of the air located in the delimited volume portion (52) after a defined minimum time after the volume portion (52) has been delimited from the environment (56), wherein the temperature data are generated by the temperature sensor (18) or are derived from analog sensor signals of the temperature sensor (18) after the temperature sensor (18) has captured the temperature of the air located in the delimited volume portion,
generating vital parameter data, wherein the vital parameter data is generated by the optical sensor device (2) or is derived from analog sensor signal data of the optical sensor device (2), wherein the vital parameter data represents at least one vital parameter of the human (4), wherein the optical sensor device (2) has captured the at least one vital parameter,
controlling a display device (58) as a function of the temperature data and/or the vital parameter data.

11. Method according to claim 10,
**characterized in that**
the optical sensor device (2) is positioned at a distance of 6 mm or less than 6 mm from the tympanic membrane (24), and
the temperature sensor (18) is positioned at a distance of 16mm or less than 16mm from the tympanic membrane (24)
and
wherein the evaluation portion (20) and the longitudinal insertion portion (12) are connected to each other via a plug connection (60) for generating a data connection, in particular a data and energy connection, wherein the optical sensor device (2) transmits the vital parameter data or vital parameter signals to the evaluation portion (20) and wherein the temperature sensor (18) transmits the temperature data or temperature signals to the evaluation portion (20)
and
wherein the device (1) for capturing at least two vital parameters, in particular the oxygen saturation and the pulse rate, is connected to a cover (62) for completely covering the ear (17) and preferably for at least partially and preferably completely covering the mastoid, wherein the cover (62) comprises the display device (58), in particular a display (58), wherein the display device (58) is controlled as a function of the vital parameter data and/or core temperature data
and
wherein the evaluation portion (20) comprises a communication interface (64) for wireless data exchange, wherein a data connection (68) is established between the communication interface (64) and a mobile terminal (66) for transmitting the vital parameter data and/or the temperature data or for transmitting evaluation data generated by the evaluation portion (20), wherein the evaluation data is generated from the vital parameter data and/or the temperature data.

## Revendications

1. Dispositif (1) pour la mesure d'au moins deux paramètres vitaux, comprenant au moins
un dispositif de capteur optique (2) pour la mesure d'au moins un paramètre vital d'un être vivant (4), dans lequel le dispositif de capteur optique (2) comprend une source de rayonnement (6) pour l'émission d'un rayonnement (7) et un détecteur de rayonnement (8) pour la détection d'un rayonnement (7), dans lequel la source de rayonnement (6) et le détecteur de rayonnement (8) sont disposés l'un à côté de l'autre,
une partie d'introduction allongée (12) pour l'introduction dans un canal auditif (14) et pour le positionnement du dispositif de capteur optique (2) dans une partie (16), entourée par des os, du canal auditif (14),
dans lequel le dispositif de capteur optique (2) est disposé sur la partie d'introduction allongée (12) ou en fait partie et dans lequel un capteur de température (18) pour la mesure de la température corporelle est disposé sur la partie d'introduction allongée (12) ou en fait partie,
une partie d'analyse (20) couplée, plus particulièrement couplée de manière amovible, avec la partie d'introduction (12), dans lequel la partie d'analyse (20) et le dispositif de capteur optique (2) sont reliés entre eux au moins temporairement, plus particulièrement dans un état couplé, pour la transmission de signaux,
dans lequel la partie d'introduction allongée (12) pouvant être introduite dans le canal auditif (14) forme, dans sa direction longitudinale (L), une extrémité interne (48) et dans lequel la partie d'introduction allongée (12) comprend ou forme un ou au moins un élément d'étanchéité (50) pour l'obturation du canal auditif (14), dans lequel le capteur de température (18) est disposé ou est réalisé entre l'extrémité interne (48) et l'élément d'étanchéité (50),
**caractérisé en ce que**
la source de rayonnement (6) est disposée ou réalisée sur une face avant (22) d'un substrat (10) pour l'émission d'un rayonnement (7) en direction d'un tympan (24) dans la direction d'allongement de la partie d'introduction (12) et
le détecteur de rayonnement (8) est disposé ou réalisé sur la face avant (22) du substrat (10) pour la détection d'un rayonnement (7) provenant du tympan (24) dans la direction d'allongement de la partie d'introduction (12),
dans lequel le substrat (10) est un PCB et
dans lequel, entre la source de rayonnement (6) et le détecteur de rayonnement (8), est réalisée une barrière de rayonnement (26),
dans lequel la source de rayonnement (6) est conçue comme au moins deux moyens d'éclairage (28, 30), plus particulièrement une première LED ou OLED et une deuxième LED ou OLED, pour l'émission d'un rayonnement (7) de différentes longueurs d'ondes.

2. Dispositif selon la revendication 1,
**caractérisé en ce que**
la partie d'introduction allongée (12) est conçue pour positionner le dispositif de capteur optique (2) à une distance inférieure ou égale à 7 mm du tympan (24), plus particulièrement du tympan (24) d'un canal auditif (14) défini, plus particulièrement à une distance entre 7 mm et 3 mm du tympan (24)
et/ou
la partie d'introduction allongée (12) présente une longueur entre 12 mm et 25 mm.

3. Dispositif selon l'une des revendications 1 à 2,
**caractérisé en ce que**
un moyen d'éclairage (28) de la source de rayonnement (6) permet d'émettre un rayonnement avec des longueurs d'ondes entre 860 nm et 900 nm, plus particulièrement entre 875 nm et 885 nm, et plus particulièrement de 880 nm,
et
dans lequel un autre moyen d'éclairage (30) de la source de rayonnement (6) permet d'émettre un rayonnement avec des longueurs d'ondes entre 620 nm et 700 nm, plus particulièrement entre 655 nm et 660 nm, et plus particulièrement de 660 nm.

4. Dispositif selon la revendication 3,
**caractérisé en ce que**
la source de rayonnement (6) comprend au moins un troisième moyen d'éclairage (31), dans lequel le troisième moyen d'éclairage (31) permet d'émettre un rayonnement entre 480 nm et 580 nm et de préférence entre 510 nm et 545 nm et plus particulièrement de préférence entre 520 nm et 535 nm,
dans lequel le détecteur de rayonnement (8) est conçu pour la détection d'un rayonnement (7) du moyen d'éclairage (28), de l'autre moyen d'éclairage (30) et du troisième moyen d'éclairage (31).

5. Dispositif selon l'une des revendications 1 à 4,
**caractérisé en ce que**
la source de rayonnement (6) et/ou le détecteur de rayonnement (8) sont entourés par un matériau (34) transparent pour le rayonnement (7) de la source de rayonnement (6) et déformable élastiquement, ou sont intégrés dans celui-ci.

6. Dispositif selon l'une des revendications 1 à 5,
**caractérisé en ce que**
le substrat (10) est entouré plusieurs fois par un matériau déformable élastiquement (34), dans lequel le matériau déformable élastiquement (34) forme au moins un évidement (36), dans lequel la source de rayonnement (6) et/ou le détecteur de rayonnement (8) sont disposés dans l'évidement (36).

7. Dispositif selon la revendication 6,
**caractérisé en ce que**
une surface (38) de la source de rayonnement (6), par l'intermédiaire de laquelle le rayonnement (7) de la source de rayonnement (6) peut être émis, est disposée à une première distance (40) du substrat (10) et dans lequel une surface externe (42) du matériau déformable élastique (34) présente, au moins dans la zone (44) entourant l'évidement (36), une deuxième distance (46) par rapport au substrat (10), dans lequel la deuxième distance (46) est soit plus grande soit égale à la première distance (40).

8. Dispositif selon la revendication 1,
**caractérisé en ce que**
la disposition du capteur de température (18) pour la détermination de la température corporelle est conçue de sorte que le capteur de température (18) peut être disposé à une distance de 9 mm et de 2 mm ou à une distance inférieure à 9 mm, plus particulièrement inférieure à 7 mm ou entre 7 mm et 2 mm et/ou à une distance d'au moins 0,1 mm du tympan (18), et dans lequel le capteur de température (18) est conçu pour la détermination de la température d'air d'un volume d'air (52) existant entre l'élément d'étanchéité (50) et l'extrémité interne (48).

9. Dispositif selon la revendication 8,
**caractérisé en ce que**
la partie d'analyse (20) et le capteur de température (18) sont reliés entre eux au moins temporairement, plus particulièrement dans l'état couplé, pour une transmission de signaux, dans lequel le capteur de température (18) est un capteur différent d'un capteur optique, dans lequel le capteur de température (18) est un thermistor, dans lequel le capteur de température est disposé ou réalisé de préférence sur une face arrière (54) du substrat (10), dans lequel la face arrière (54) est réalisée de préférence de manière parallèle ou de préférence de manière parallèle à certains endroits, par rapport à la face supérieure (32).

10. Procédé de mesure de paramètres vitaux d'un être humain (4), comprenant au moins les étapes suivantes :
mise à disposition d'un dispositif (1) pour la mesure d'au moins deux paramètres vitaux selon l'une des revendications précédentes,
introduction de la partie d'introduction allongée (12) dans un canal auditif (14) d'un être humain (4),
positionnement du dispositif de capteur optique (2) à une distance de 7 m ou inférieure à 7 mm du tympan (24),
positionnement du capteur de température (18) à une distance de 16 mm ou inférieure à 16 mm du tympan (24),
délimitation d'une partie de volume (52), contenant de l'air, du canal auditif (14), au moyen d'un élément d'étanchéité (50) de la partie d'introduction allongée (12) par rapport à l'environnement (56),
génération de données de température, plus particulièrement de données de température centrales, dans lequel les données de température représentent la température de l'air se trouvant dans la partie de volume (52) délimitée, après une durée minimale définie après que la partie de volume (52) a été délimitée par rapport à l'environnement (56), dans lequel les données de température sont générées par le capteur de température (18) ou sont déduites de signaux de capteurs analogiques du capteur de température (18) après que le capteur de température (18) a mesuré la température de l'air se trouvant dans la partie de volume délimitée,
génération de données de paramètres vitaux, dans lequel les données de paramètres vitaux sont générées par le dispositif de capteur optique (2) ou sont déduites de signaux de capteurs analogiques du dispositif de capteur optique (2), dans lequel les données de paramètres vitaux représentent au moins un paramètre vital de l'être humain (4), dans lequel le dispositif de capteur optique (2) a mesuré l'au moins un paramètre vital,
contrôle d'un dispositif d'affichage (58) en fonction des données de température et/ou des données de paramètres vitaux.

11. Procédé selon la revendication 10,
**caractérisé en ce que**
le dispositif de capteur optique (2) est positionné à une distance de 6 mm ou inférieure à 6 mm du tympan (24) et
le capteur de température (18) est positionné à une distance de 16 mm ou inférieure à 16 mm du tympan (24),
et
dans lequel la partie d'analyse (20) et la partie d'introduction allongée (12) sont reliées entre elles par l'intermédiaire d'une liaison enfichable (60) pour la génération d'une liaison de données, plus particulièrement une liaison de données et d'énergie, dans lequel le dispositif de capteur optique (2) transmet les données de paramètres vitaux ou les signaux de paramètres vitaux à la partie d'analyse (20) et dans lequel le capteur de température (18) transmet les données de température ou les signaux de température à la partie d'analyse (20),
et
dans lequel le dispositif (1) pour la mesure d'au moins deux paramètres vitaux, plus particulièrement la saturation en oxygène et la fréquence de pulsation, est relié avec un recouvrement (62) pour le recouvrement complet de l'oreille (17) et de préférence pour le recouvrement au moins partiel et de préférence complet du mastoïde, dans lequel le recouvrement (62) comprend le dispositif d'affichage (58), plus particulièrement un écran (58), dans lequel le dispositif d'affichage (58) est contrôlé en fonction des données de paramètres vitaux et/ou des données de température centrale
et
dans lequel la partie d'analyse (20) comprend une interface de communication (64) pour l'échange de données sans fil, dans lequel, entre l'interface de communication (64) et un terminal mobile (66), une liaison de données (68) est établie pour la transmission des données de paramètres vitaux et/ou des données de température ou pour la transmission des données d'analyse générées par la partie d'analyse (20), dans lequel les données d'analyse sont générées à partir des données de paramètres vitaux et/ou des données de température.
